(19) Europäisches Patentamt
European Patent Office
Office européen des brevets

(11) Veröffentlichungsnummer: **0 210 474**
**B1**

(12) **EUROPÄISCHE PATENTSCHRIFT**

(45) Veröffentlichungstag der Patentschrift: **29.08.90**

(21) Anmeldenummer: **86109211.2**

(22) Anmeldetag: **05.07.86**

(51) Int. Cl.⁵: **C 07 D 271/04**, C 07 D 413/12, C 07 D 493/04, A 61 K 31/41

# (54) Substituierte 3-Amino-sydnonimine, Verfahren zu ihrer Herstellung, ihre Verwendung und sie enthaltende pharmazeutische Präparate.

(30) Priorität: **20.07.85 DE 3526068**

(43) Veröffentlichungstag der Anmeldung:
**04.02.87 Patentblatt 87/06**

(45) Bekanntmachung des Hinweises auf die Patenterteilung:
**29.08.90 Patentblatt 90/35**

(84) Benannte Vertragsstaaten:
**AT BE CH DE FR GB IT LI NL SE**

(56) Entgegenhaltungen:
**EP-A-0 076 952**
**EP-B-0 024 924**
**DE-A-2 930 736**
**DE-A-3 107 933**

**CHEMICAL ABSTRACTS, Band 91, Nr. 13, 24. September 1979, Columbus, Ohio, USA; NITZ, R.E. "Pharmacology of molsidomine." Seite 5, Spalte 2, Zusammenfassung-Nr. 101707v**

**Die Akte enthält technische Angaben, die nach dem Eingang der Anmeldung eingereicht wurden und die nicht in dieser Patentschrift enthalten sind.**

(73) Patentinhaber: **CASSELLA Aktiengesellschaft**
**Hanauer Landstrasse 526**
**D-6000 Frankfurt am Main 61 (DE)**

(72) Erfinder: **Schönafinger, Karl, Dr.**
**Holunderweg 8**
**D-8755 Alzenau (DE)**
Erfinder: **Beyerle, Rudi, Dr.**
**An der Pfaffenmauer 44**
**D-6000 Frankfurt am Main (DE)**
Erfinder: **Bohn, Helmut, Dr.**
**Kranzbergring 11**
**D-6369 Schöneck (DE)**
Erfinder: **Just, Melitta, Dr.**
**Hüttenweg 6**
**D-6369 Schöneck (DE)**
Erfinder: **Martorana, Piero, Dr.**
**Kaiser-Friedrich-Promenade 108**
**D-6380 Bad Homburg (DE)**
Erfinder: **Nitz, Rolf-Eberhard, Dr.**
**Heinrich Bingemer Weg 64**
**D-6000 Frankfurt am Main (DE)**

(74) Vertreter: **Urbach, Hans-Georg, Dr.**
**Hanauer Landstrasse 526**
**D-6000 Frankfurt am Main 61 (DE)**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

Courier Press, Leamington Spa, England.

EP 0 210 474 B1

(56) Entgegenhaltungen:
CHEMICAL Abstracts, Band 91, Nr. 15, 8. Oktober 1979, Columbus, Ohio, USA; FLECKENSTEIN-GRUEN, G.; FLECKENSTEIN, A.; SPAEH, F.; ASSMANN, R.

"Electromechanical coupling process in isolated cardiac and vascular musculature effected by molsidomine and its metabolite SIN-1". Seite 4, Spalte 2, Zusammenfassung-Nr. 117o35k

**Beschreibung**

Die Erfindung betrifft das substituierte 3-Aminosydnonimin der Formel I

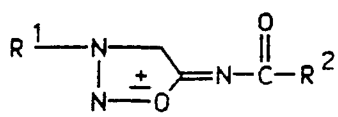

(I)

in optisch aktiver Form und seine pharmakologisch annehmbaren Säureadditionssalze, worin

$R^1$ den Rest

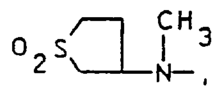

$R^2$ den Rest 2-Bornyloxy bedeuten.

Die Erfindung betrifft ferner ein Verfahren zur Herstellung der Verbindung der Formel I, ihre Verwendung und sie enthaltende pharmazeutische Präparate.

Der für $R^2$ stehende Rest enthält asymmetrische Kohlenstoffatome. Dadurch liegt die Verbindung der Formel I in optisch aktiver (R)- oder (S)-Form ((R = "rectus", (S) = "sinister") vor.

Das substituierte 3-Aminosydnonimin der Formel I wird durch Acylierung des 3-Aminosydnonimins der Formel III

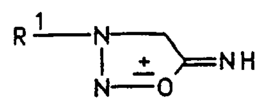

(III)

oder dessen Salzen mit Acylierungsmitteln, die den Acylrest $R^2$—CO— einführen, hergestellt. Bei dieser Acylierung wird ein Wasserstoffatom der Iminogruppe der Verbindung III durch den Rest $R^2$—CO-ersetzt. Geeignete Acylierungsmittel sind z.B. Verbindungen der Formel IV

$$R^2\text{—CO—X} \qquad \text{(IV)}$$

worin $R^2$ die bereits genannte Bedeutung besitzt und X=Halogen, insbesondere —Cl oder —Br, —OH, —O-Alkyl, insbesondere mit 1 bis 5 C-Atomen, —O—CO—$R^2$ oder —O—CO—O-Alkyl, insbesondere mit 1 bis 5 C-Atomen im Alkylrest, —O-Aryl, —O-Nitroaryl oder —O-Dinitroaryl, insbesondere Phenoxy, 2- oder 4-Nitrophenoxy oder 2,4-Dinitrophenoxy, —$OCH_2CN$ oder den über ein N-Atom gebundenen Rest eines Azols oder Benzazols mit mindestens 2 N-Atomen im quasiaromatischen Fünfring bedeutet.

Die Acylierungsmittel der Formel IV stellen somit z.B. Carbonsäurehalogenide, insbesondere das Carbonsäurechlorid und das Carbonsäurebromid, von denen das Carbonsäurechlorid bevorzugt ist, die Carbonsäure, Carbonsäureester, das Carbonsäureanhydrid oder gemischt Carbonsäure-Kohlensäure-Anhydride oder heterocyclische Amide oder Azolide dar.

Bei der Acylierung brauchen die Acylierungsmittel der Formel IV, wobei X die angegebene Bedeutung mit Ausnahme von —OH besitzt, nicht unbedingt in reiner Form eingesetzt zu werden, sondern sie können auch kurz vor der Acylierungsreaktion oder während der Acylierungsreaktion in situ aus der Carbonsäure der Formel, IVa

$$R^2\text{—CO—OH} \qquad \text{(IVa)}$$

erzeugt werden. Das heißt, daß als Acylierungsmittel auch die Carbonsäure der Formel IVa verwendet werden kann.

Falls als Acylierungsmittel die Carbonsäure der Formel IVa verwendet wird, ist der Zusatz eines Aktivierungsmittel zweckmäßig, das die Aufgabe hat, das Acylierungspotential der Carbonsäure zu erhöhen bzw. zu aktivieren. Als derartige Aktivierungsmittel sind z.B. geeignet:

a) wasserentziehende bzw. wasserbindende Mittel und

b) solche Agenzien, welche die Carbonsäure der Formel IVa in die entsprechenden, als Acylierungsmittel wirkenden Säurehalogenide, Anhydrid, Ester, gemischte Carbonsäure-Kohlensäure-Anhydride oder Azolide zu überführen vermögen.

Als geeignete wasserbindende bzw. wasserentziehende Mittel kommen z.B. N,N'-disubstituierte Carbo-diimide der Formel V

$$R'\text{—N=C=N—}R'' \qquad \text{(V)}$$

wobei R' und R'' aliphatische, cycloaliphatische, araliphatische, aromatische oder heteroaromatische Reste darstellen, in Betracht, insbesondere dann, wenn der Rest R' und gegebenenfalls auch der Rest R'' ein sekundäre oder tertiärer Alkylrest ist (vgl. Methodicum Chimicum, Verlag G. Thieme Stuttgart, Bd. 6, (1974), S.682). Geeignete Carbo-diimide sind z.B. Di-ispropyl-, Di-cyclohexyl-oder Methyl-tert.butyl-carbo-diimid. Bei der Durchführung der Acylierungsreaktion werden dann die Verbindung der Formel III und die Carbonsäure der Formel IVa und das Carbo-diimid in einem geeigneten inerten Lösungs- oder Verdünnungsmittel zusammengegeben, wodurch sich das gewünschte Acylierungsprodukt der Formel I und aus dem Carbo-diimid der entsprechende disubstituierte Harnstoff bildet.

Beispiele für Agenzien, welche die Carbonsäure der Formel IVa in die entsprechenden Halogenide, Carbonester, Anhydrid, gemischte Carbonsäure-Kohlensäure-Anhydride oder Azolide überführen können, sind vor allem Kohlensäurederivate, wie z.B. Phosgen Cl—CO—Cl, Chlorameisensäureester Cl—CO—O-Alkyl, insbesondere mit 1 bis 5 C-Atomen im Alkylrest (vgl. z.B. Tetrahedron Letters *24* (1983) 3365), Kohlensäureester R'''—O—CO—O—R'''', wobei R''' und R'''' aromatische oder heteroaromatische Reste oder über das N-Atom gebundene Reste von cyclischen Säureimiden aliphatischer oder aromatischer Carbonsäuren darstellen, wie z.B. N,N'-Disuccinimido-carbonat, Diphthalimido-carbonat, 1,1'-(Carbonyldioxy)-di-(benzo-triazol) oder Di-2-pyridylcarbonat (vgl. z.B. Tetrahedron Letters, Vol. 25, No. 43, 4943—4946), gegebenenfalls in Anwesenheit geeigneter Katalysatoren, wie z.B. 4-Dimethylaminopyridin, oder heterocylische Diamide der Kohlensäure der Formel A—CO—A, worin A ein über ein N-Atom gebundener Rest eines Azols mit mindestens 2 Stickstoffatomen im quasi-aromatischen Fünfring bedeutet. Geeignete derartige heterocylische Diamide sind z.B. N,N'-Carbonyl-diimidazol, 2,2'-Carbonyl-ditriazol(1,2,3), 1,1'-Carbonyl-ditriazol(1,2,4), N,N'-Carbonyl-dipyrazol, 2,2'-Carbonyl-ditetrazol, N,N'-Carbonyl-benzimidazol oder N,N'-Carbonylbenztriazol. Diese Verbindungen werden im allgemeinen vor der eigentlichen Acylierung der Verbindung III mit der Carbonsäure der Formel IVa in einem geeigneten Lösungs-oder Dispergierungsmittel in stöchiometrischen Verhältnissen bei Temperaturen von 0°C bis zum Siedepunkt des Lösungs-oder Verdünnungsmittels, normalerweise bei 10 bis 100°C, vorzugsweise 20 bis 80°C, vereinigt, wobei sich in wenigen Minuten das als eigentliches Acylierungsmittel wirkende Azolid der Formel

$$R^2\text{—CO—A}$$

bildet, wobei $R^2$ und A die bereits genannten Bedeutungen besitzen. Dieses kann dann sofort im gleichen Topf zur Acylierung des 3-Aminosydnonimins der Formel III verwendet werden (vgl. z.B. H. A. Staab, M. Lückung u. F. H. Dürr, Chem. Ber. *95*, 1962), 1275, H. A. Staab und W. Rohr "Synthesen mit heterocyclischen Amiden (Azoliden)" in "Neuere Methoden der Präparativen Organischen Chemie", Band V, Verlag Chemie, 1967, S.53, ff., insbesondere S.68). Als N,N'-Carbonyl-diazol wird häufig das käufliche N,N'-Carbonyl-diimidazol verwendet. Die anderen N,N'-Carbonylazole sind aber aus dem jeweiligen Azol und Phosgen ebenfalls leicht zugänglich.

Als Aktivierungsmittel für die Carbonsäure der Formel IVa können anstelle der Kohlensäurederivate häufig auch die entsprechenden Derivate der Oxalsäure, wie z.B. Oxalylchlorid Cl—CO—CO—Cl (vgl. z.B. GB—PS 2 139 725) oder N,N-Oxalyl-diazole A—CO—CO—A, wobei A die bereits genannte Bedeutung besitzt (vgl. z.B. Bull Chem. Soc. Jap. *57*, 3597—3598 (1984)) eingesetzt werden.

Als Aktivierungsmittel für die Carbonsäure IVa sind jedoch auch andere Verbindungen, wie z.B. Methylethylphosphinsäureanhydrid (vgl. z.B. DE—OS 31 01 427) geeignet.

Die Umsetzung zwischen dem Acylierungsmittel und der Verbindung III wird zweckmäßigerweise in flüssiger Phase in Anwesenheit eines inerten Lösungs-, Dispergier- oder Verdünnungsmittels durchgeführt.

Geeignete Lösungs-, Dispergier- oder Verdünnungsmittel sind z.B. Alkohole, insbesondere solche mit 1 bis 6 C-Atomen wie z.B. Methanol, Ethanol, i- und n-Propanol, i-, sec- und tert-Butanol, n-, i-, sec-, tert-Pentanol, n-Hexanol, Cyclopentanol, Cyclohexanol; Ether, insbesondere solche mit 2 bis 8 C-Atomen im Molekül, wie z.B. Diethylether, Methyl-ethyl-ether, Di-n-propyl-ether, Di-isopropyl-ether, Methyl-n-butyl-ether, Ethylpropyl-ether, Di-butyl-ether, Tetrahydrofuran; 1,4-Dioxan, 1,2-Dimethoxyethan, Bis-β-methoxy-ethyl-ether; Polyether, wie. z.B. Polyethylenglykole mit einem Molekulargewicht bis ca. 600; Oligoethylenglycol-dimethyl-ether, wie z.B. Pentaglyme; Kronenether, d.h. cyclische Polymere des Ethylenglykols der Formel $(\text{—}OCH_2CH_2)_p$, wobei p eine Zahl z.B. von 4 bis 10 ist, wobei an den Ring auch eine oder mehrere Benzolringe ankondensiert sein können; Aza- und Thia-kronenether (Coronand-amine und Coronand-sulfide); Glykole und teilweise veretherte Glykole, wie z.B. Ethylenglykol, Propylenglykol, Trimethylenglykol, Ethylenglykol-monomethyl-ether, Ethylenglykol-monoethyl-ether, Diethylenglykolmonoethyl-ether; aliphatische Kohlenwasserstoffe, wie z.B. Benzine, niedrig- und hochsiedende Petrolether; aromatische Kohlenwasserstoffe, wie z.B. Benzol, Toluol, o-, m- und p-Xylol, Pyridin; halogenierte aliphatische oder aromatische Kohlenwasserstoffe, wie z.B. Methylenchlorid, Chloroform, Tetrachlorokohlenstoff, Ethylenchlorid, Chlorbenzol, Dichlobenzol; Nitrile, wie z.B. Acetonitril; Amide, wie z.B. Dimethylformamid, N-Methyl-pyrrolidon; Sulfoxide, wie z.B. Dimethyl-sulfoxid; Wasser. Auch Gemische verschiedener Lösungs-, Dispergier- oder Verdünngsmittel können verwendet werden, z.B. Wasser/Methylenchlorid, Wasser/Toluol. Auch ein Überschuß des Acylierungsmittels kann als Lösungs-, Dispergier- oder Verdünnungsmittel verwendet werden.

Die als Lösungs-, Dispergier- oder Verdünnungsmittel angegebenen Alkohole, Glykole und teilweise

veretherten Glykole sowie Wasser sind normalerweise nur für die Acylierung mit Carbonsäureestern geeignet, dagegen für die Durchführung der Acylierung mit anderen Acylierungsmitteln wegen der konkurierenden Bildung von Estern, Glykolestern oder Säuren nicht ausreichend inert und daher weniger geeignet.

Das Molverhältnis zwischen der Verbindung der Formel III und dem Acylierungsmittel beträgt 1:1. Zweckmäßigerweise wird das Acylierungsmittel in einem geringen molaren Überschuß eingesetzt. Überschüsse von bis zu 30 mol% sind in der Regel ausreichend, d.h. das Molverhältnis zwischen der Verbindung der Formel III und dem Acylierungsmittel der Formel IV beträgt normalerweise 1: (1 bis 1,3), vorzugsweise 1: (1 bis 1,2). Falls bei der Acylierungsreaktion eine Säure abgespalten wird, ist der Zusatz eines Säurefängers, wie z.B. eines Alkalihydroxids, wie z.B. Natrium-, Kalium- oder Lithium-hydroxids, eines tertiären organischen Amins, wie z.B. Pyridin oder Triethylamin, eines Alkalicarbonats oder Alkalibicarbonats, wie z.B. Soda oder Natriumbicarbonat, oder eines Alkalisalzes einer schwachen organischen Säure, wie z.B. Natriumacetat, zweckmäßig. Bei der Acylierungsreaktion können auch geeignete Katalysatoren, wie z.B. 4-Dimethylaminopyridin, zugesetzt werden.

Die Umsetzung zwischen dem Acylierungsmittel und der Verbindung III kann prinzipiell bei Temperaturen zwischen −10°C und den Siedepunkt des verwendeten Lösungs-, Dispergier- oder Verdünnungsmittels erfolgen. In vielen Fällen wird die Umsetzung bei 0 bis 50°C, insbesondere bei 0 bis 30°C und vorzugsweise bei Raumtemperatur, durchgeführt.

Die als Acylierungsmittel eingesetzten Carbonsäurederivate besitzen mindesetns ein Asymmetriezentrum. Bei der Acylierung mit enantiomerenreinen bzw. optisch aktiven Acylierungsmitteln der Formel IV erhält man direkt die optisch aktiven Verbindungen der Formel I. Dieses Verfahren ist bevorzugt. Bei der Acylierung mit Acylierungsmitteln der Formel IV, die Racemate darstellen, erhält man zunächst das Racemat, das in an sich bekannter Weise, z.B. durch Überführung in diastereomere Salze in die optisch aktiven Verbindungen der Formel I zerlegt werden kann.

Das substituierte 3-Amino-sydnonimin der Formel I bildet mit anorganischen oder organischen Säuren Säureadditionssalze. Zur Bildung derartiger Säureadditionssalze sind anorganische oder organische Säuren geeignet. Geeignete Säuren sind beispielsweise Chlorwasserstoff Bromwasserstoff, Naphthalindisulfonsäure, insbesondere, 1,5-Napthalindisulfonsäure, Phosphor-, Salpeter-, Schwefel-, Oxal-, Milch-, Wein-, Essig-, Salicyl-, Benzoe-, Ameisen-, Propion-, Pivalin-, Diethylessig-, Malon-, Bernstein-, Pimelin-, Fumar-, Malein-, Apfel-, Sulfamin-, Phenylpropion-, Glucon-, Ascorbin-, Isonicotin-, Methansulfon-, p-Toluolsulfon-, Zitronen- oder Adipin-Säure. Pharmakologisch annehmbare Säureadditionssalze werden bevorzugt. Die Säureadditionssalze können wie üblich durch Vereinigung der Komponenten, zweckmäßigerweise in einem geeigneten Lösungs- oder Verdünnungsmittel, hergestellt werden. Falls bei der Synthese der Verbindung der Formel I die Säureadditionssalze anfallen, kann die freie Verbindung der Formel I aus den Säureadditionssalzen gewünschtenfalls in bekannter Weise, d.h. durch Auflösen oder Suspendieren in Wasser oder Alkalischstellen, z.B. mit Natronlauge, und anschließendes Isolieren, gewonnen werden.

Die benötigte Ausgangsverbindung der allgemeinen Formel III ist bekannt (vgl. z.B. EP 59 356) bzw. kann nach an sich bekannten Methoden hergestellt werden.

Auch die Acylierungsmittel der Formel IV bzw. IVa sind bekannt bzw. können nach den für die jeweilige Substanzklasse bekannten Methoden hergestellt werden.

Die Verbindung der Formel I und ihre pharmakologisch annehmbaren Säureadditionssalze besitzen wertvolle pharmakologische Eigenschaften.

Besonders ausgeprägt ist ihre Wirkung auf das Herz-Kreislauf-System. Sie senken beispielsweise den Blutdruck ebenso wie den Pulmonalartieriendruck und den linksventrikulären enddiastolischen Druck und tragen so zu einer Entlastung der Herztätigkeit im Sinne einer antianginösen Wirkung bei, ohne dabei, eine reflektorische Tachykardie zu provozieren. Gegenüber ähnlich gebauten Verbindungen, insbesondere gegenüber der im Handel befindlichen Verbindung Molsidomin (=3-Morpholino-N-ethoxycarbonyl-sydnonimin) und den Verbindungen gemäß den euroäpischen Patentschriften 0023343 und 0059356 besitzen sie vor allem eine deutliche längere Wirkung, was für die Applikation und Compliance eine große Bedeutung besitzt.

Die Verbindungen der europäischen Patentschrift 0023 343 sind substituierte 3-Amino-sydnonimine der Formel

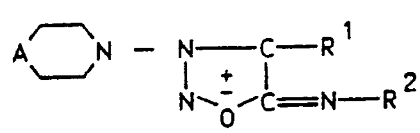

und ihre pharmakologisch annehmbaren Säureadditionssalze, worin

$R^1$ Wasserstoff oder Halogen,

$R^2$ Wasserstoff, —NO oder eine —$COR^3$ oder —$SO_2R^4$-Gruppe,

$R^3$ Wasserstoff, Alkyl mit 1 bis 6 C-Atomen, das auch durch Alkoxy mit 1 bis 6 C-Atomen oder Aryloxy mit 6 bis 12 C-Atomen substituiert sein kann, Cycloalkyl mit 5 bis 8 C-Atomen, Aryl mit 6 bis 12 C-Atomen, das auch durch Halogen und/oder Alkyl mit 1 bis 4 C-Atomen und/oder Alkoxy mit 1 bis 4 C-Atomen mono-,

di- oder trisubstituiert sein kann, Aralkyl oder Aralkenyl mit 7 bis 13 C-Atomen, Alkoxy mit 1 bis 6 C-Atomen, Aryloxy mit 6 bis 12 C-Atomen, Pyridyl, Pyrrolyl, Imidazolyl, Pyrazolyl, Pyrazinyl, Pyrimidinyl, Pyridazinyl, Alkoxycarbonyl mit insgesamt 2 bis 7 C-Atomen,

A die Gruppe

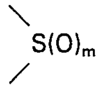 ,

wobei m = 0, 1 oder 2 ist, oder die Gruppe

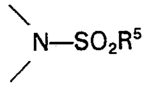 ,

$R^4$ und $R^5$ unabhängig voneinander Alkyl mit 1 bis 6 C-Atomen, Aryl mit 6 bis 12 C-Atomen, das auch durch Methyl oder Chlor substituiert sein kann, oder eine Dialkylaminogruppe mit 1 bis 4 C-Atomen in jedem der Alkylreste bedeuten.

Die Verbindungen der europäischen Patentschrift 0 059 356 sind substituierte 3-Amino-sydnonimine der allgemeinen Formel

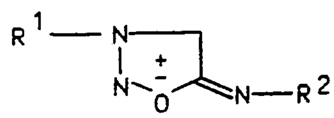

und ihre pharmakologisch annehmbaren Säureadditionssalze, worin $R^1$ den Rest

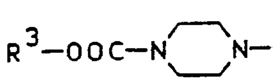 oder den Rest 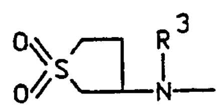

$R^2$ Wasserstoff oder den Rest —CO—$R^4$,

$R^3$ Methyl, Ethyl, Propyl, Isopropyl,

$R^4$ einen aliphatischen Rest mit 1 bis 4 C-Atomen, der auch durch Alkoxy mit 1 bis 3 C-Atomen substituiert sein kann, einen cycloaliphatischen Rest mit 5 bis 7 C-Atomen, einen bicycloaliphatischen Rest mit 7 bis 14 C-Atomen, einen tricycloaliphatischen Rest mit 7 bis 16 C-Atomen, einen Alkoxyrest mit 1 bis 6 C-Atomen, einen Aryloxyrest mit 6 bis 12 C-Atomen oder einen Alkoxycarbonylrest mit insgesamt 2 bis 7 C-Atomen, einen Arylrest mit 6 bis 12 C-Atomen, einen durch 1 bis 3 Halogenatome und/oder 1 bis 3 Alkylreste mit 1 bis 3 C-Atomen und/oder 1 bis 3 Alkoxyreste mit 1 bis 3 C-Atomen und/oder 1 oder 2 Nitrogruppen mono-, di- oder trisubstituierten Arylrest mit 6 bis 12 C-Atomen bedeutet.

Die Verbindung der Formel I und ihre pharmakologisch annehmbaren Säureadditonssalze können daher am Menschen als Heilmittel für sich allein, in Mischungen untereinander oder in Form von pharmazeutischen Zubereitungen verabreicht werden, die eine enterale oder parenterale Anwendung gestatten und die als aktiven Bestandteile eine wirksame Dosis mindestens einer Verbindung der Formel I oder eines Säureadditionssalzes davon neben üblichen pharmazeutisch einwandfreien Träger- und Zusatzstoffen enthalten.

Die Heilmittel können oral, z.B. in Form von Pillen, Tabletten, Lacktabletten, Dragees, Hart- und Weichgelatinekapsel, Lösungen, Sirupen, Emulsionen oder Suspensionen oder Aerosolmischungen verabreicht werden. Die Verabreichung kann aber auch rektal, z.B. in Form von Suppositorien, oder parenteral, z.B. in Form von Injektionslösungen, oder perkutan, z.B. in Form von Salben oder Tinkturen, erfolgen.

Zur Herstellung der pharmazeutischen Präparate können pharmazeutische inerte anorganische oder organische Trägerstoffe verwendet werden. Für die Herstellung von Pillen, Tabletten, Dragees und Hartgelatinekapseln kann man z.b. Lactose, Maisstärke oder Derivate davon, Talk, Stearinsäure oder deren Salze etc. verwenden. Trägerstoffe für Weichgelatinekapseln und Suppositorien sind z.B. Fette, Wachse, halbfeste und flüssige Polyole, natürliche oder gehärtete Öle etc. Als Trägerstoffe für die Herstellung von Lösungen und Sirupen eignen sich z.B. Wasser, Saccharose, Invertzucker, Glukose, Polyole etc. Als Trägerstoffe für die Herstellung von Injektionslösungen eignen sich z.B. Wasser, Alkohole, Glyzerin, Polyole, pflanzliche Öle etc.

Die pharmazeutische Präparate können neben den Wirk- und Trägerstoffen noch Zusatzstoffe, wie. z.B. Füllstoffe, Streck-, Spreng-, Binde-, Gleit-, Netz-, Stabiliserungs-, Emulgier-, Konservierungs-, Süß-, Färbe-, Geschmacks- oder Aromatisierungs-, Dickungs-, Verdünnungs-Mittel, Puffersubstanzen, ferner

Lösungsmittel oder Lösungsvermittler oder Mittel zur Erzielung eines Depoteffekts, sowie Salze zur Veränderung des osmotischen Drucks, Überzugsmittel oder Antioxidantien enthalten. Sie können auch zwei oder mehrere pharmakologisch annehmbaren Säureadditionssalze und noch andere therapeutisch wirksame Stoffe enthalten.

Derartige andere therapeutisch wirksame Substanzen sind beispielsweise: β-Rezeptorenblocker, wie z.B. Propranolol, Pindolol, Metroprolol; Vasodilatatoren, wie z.B. Carbochomen, antianginöse Mittel, wie z.B. Carbochromen oder Molsidomin; Beruhigungsmittel, wie z.B. Barbitursäurederivate, 1,4-Benzodiazepine und Meprobamat; Diuretica, wie z.B. Chlorothiazid; das Herz tonisierende Mittel, wie z.B. Digitalispräparate; blutdrucksenkende Mittel, wie z.B. Hydralazin, Dihydralazin, Prazosin; Clonidin, Rauwolfia-Alkaloide; Mittel, die den Fettsäurespiegel im Blut senken, wie z.B. Bezafibrat, Fenofibrat; Mittel für die Thromboseprophylaxe, wie z.B. Phenprocoumon.

Die Verbindung der Formel I, ihre pharmakologisch annehmbaren Säureadditionssalze und pharmazeutische Präparate, welche die Verbindung der Formel I oder ihre pharmakologisch annehmbaren Säureadditionssalze als Wirkstoff enthalten, können am Menschen bei der Bekämpfung bzw. Vorbeugung von Erkrankungen verwendet werden, beispielsweise als antihypertensive Heilmittel bei den verschiedenen Formen des Bluthochdrucks, bei der Bekämfung bzw. Vorbeugung von Angina pectoris usw., sowie bei der Behandlung von cerebralen und peripheren Durchblutungsstörungen eingesetzt werden. Die Dosierung kann innerhalb weiter Grenzen variieren und ist in jedem einzelnen Fall den individuellen Gegebenheiten anzupassen. Im allgemeinen ist bei oraler Verabreichung eine Tagesdosis von etwa 0,01 bis 10 mg/kg, vorzugsweise 0,05 bis 5 mg/kg, Körpergewicht zur Erzielung wirksamer Ergebnisse angemessen. Bei intravenöser Applikation beträgt die Tagesdosis im allgemeinen etwa 0,001 bis 120 mg/kg, vorzugsweise 0,01 bis 5 mg/kg, Körpergewicht. Die Tagesdosis wird normalerweise, insbesondere bei der Applikation größerer Mengen, in mehrere, z.B. 2, 3 oder 4, Teilverabzeichungen aufgeteilt. Gegebenenfalls kann es je nach individuellem Verhalten erforderlich werden, von der angegebenen Tagesdosis nach oben oder unter abzuweichen.

Bei dem in den nachfolgenden Beispielen angegebenen Wert $\alpha_D^{20}$ handelt es sich um die spezifische Drehung der Substanz für polarisiertes Licht der Natrium-D-Linie (589 nm) bei 20°C. Nach dem angegebenen Wert für die spezifische Drehung sind in den Beispielen in Klammern das bei der Messung benutzte Lösungsmittel und die Konzentration c in g/100 ml angegeben. Falls entsprechende Angaben fehlen, sind die Messungen in Methanol bei einer Konzentration von 5,0 g in 100 ml Lösung durchgeführt worden.

## Beispiel 1

a) (S)-(−)-Chlorameisensäure-(2-methyl-butylester)

50 g Phosgen werden unter guter Kühlung in 250 ml Methylenchlorid gelöst. Zu dieser kalten Lösung werden 22 g (S)-(−)-2-Methyl-1-butanol getropft. Die Lösung läßt man dann die Raumtemperatur erreichen, rührt über Nacht nach, engt ein und destilliert den Rückstand im Vakuum.

Ausbeute: 32,6 g; $Kp_{20mbar}$ = 56 bis 58°C.

b) (S)-(+)-N-(2-Methyl-butoxycarbonyl)-3-(4-methylsulfonyl-piperazin-1-yl)-sydnonimin

5 g 3-(4-Methylsulfonyl-piperazin-1-yl)-sydnonimin-hydrochlorid werden in 25 ml $H_2O$ suspendiert und auf 5°C abgekühlt. 3,2 g Natriumhydrogencarbonat werden zugefügt, und die Lösung von 3 g (S)-Chlorameisensäure-(2-methyl-butyl-ester) in 25 ml Methylenchlorid wird rasch unter gleichzeitigem Umrühren zugetropft. Die Mischung wird 6 h nachgerührt, wobei man auf Raumtemperatur erwärmen läßt. Dann wird die Methylendchloridphase abgetrennt, über $Na_2SO_4$ getrocknet und eingeengt. Der Rückstand wird aus Ethanol umkristallisiert.

Ausbeute: 5,1 g; Fp = 113 bis 115°C; $\alpha_D^{20}$ = +4,0°.

Analog Beispiel 1 läßt sich herstellen.

## Beispiel 2

(−)-(2-Bornyl)-oxycarbonyl-3-(N-methyl-N-(sulfolan-3-yl-1,1-dioxo)-amino)-sydnonimin.

Fp = 157°C (Zers.); $\alpha_D^{20}$ = −20°.

Die folgenden Beispiele betreffen pharmazeutische Zubereitungen.

Beispiel A

| Tabletten | pro Tablette |
|---|---|
| Wirkstoff | 20 mg |
| Lactose | 60 mg |
| Maisstärke | 30 mg |
| lösliche Stärke | 5 mg |
| Magnesiumstearat | 5 mg |
| | 120 mg |

Beispiel B

| Dragees | |
|---|---|
| Wirkstoff | 6 mg |
| Propanolol | 40 mg |
| Milchzucker | 90 mg |
| Maisstärke | 90 mg |
| sec.Calciumphosphat | 34 mg |
| | 260 mg |

Beispiel C

| Kapseln | |
|---|---|
| Wirkstoff | 55 mg |
| Prazosin | 5 mg |
| Maisstärke | 185 mg |
| | 195 mg |

Beispiel D

| Injektionslösung | |
|---|---|
| Wirkstoff | 4 mg |
| Natriumchlorid | 0,7 mg |
| Wasser zu Injektionszwecken ad | 1 ml |

Beispiel E

| Suppositorien | |
|---|---|
| Wirkstoff | 20 mg |
| Suppositoriumsgrundmasse ad | 2 g |

Die pharmakologische Wirksamkeit der erfindungsgemäßen Verbindungen wurde nach der folgenden Methode geprüft:

Die Untersuchungen wurden an Bastardhunden beiderlei Geschlechts mit einem Gewicht von 15 bis 25 kg vorgenommen. Nach intravenöser Vorbehandlung mit 22 mg Piritramid (Dipidolor®) wurden dei Tiere mit Natriumpentobarbital (30 mg/kg i.v.) narkotisiert und künstlich beatmet. Zur Blutdruckmessung wurde ein mit Flüssigkeit gefüllter Tygon-Katheter (Durchmesser 1,0 × 1,78 mm) über die oberflächliche zirkumflexe Arteria ilica in die Aorta abdominalis eingeschoben. Am fünften Zwischenrippenraum wurde ein linksseitiger Brustschnitt vorgenommen, und in den linken Ventrikel wurde zur Messung des linksventrikulären Druckes und der Kontraktilität (BD, LVEDP, dp/dt) ein dickwandiger Tygon-Katheter

(Durchmesser 0,76 × 2,28 mm) eingeschoben. Der Hochdruck wurde durch Anlegen von Konstriktoren um die Arteriae renales auf beiden Seiten induziert.

Hämodynamisches Messungen wurden erst 14 Tage nach dem Eingriff vorgenommen. Die zu prüfenden Substanzen wurden oral in einer Dosis von 0,2 mg/kg verabreicht.

Es wurden die in der nachfolgenden Tabelle angegebenen Werte erhalten:

TABELLE

| Verbindung gem.Beisp. | Δ BD s/d (mm Hg) | Δ LVEDP (mm Hg) | Δ HF (S/min) | Δ dp/dt (mm Hg/s) |
|---|---|---|---|---|
| 2 | −25/−5 360′ | −5 300′ | +35 | −200 |
| Molsidomin (Vergleichssubstanz) | −35/−10 20′ | −6 120′ | +20 | −200 |

Die in der vorstehende Tabelle in den einzelnen Spalten angegebenen Werte haben folgende Bedeutungen:

a) Δ BD s/d (mm Hg): Änderung des Blutdrucks systolisch/diastolisch in mm Hg. Die in dieser Spalte angegebene dritte Zahl gibt die Dauer des Effektes in Minuten an.

b) Δ LVEDP (mm Hg): Änderung des linksventrikulären enddiastolischen Drucks in mm Hg. Die in dieser Spalte angegebene zweite Zahl gibt die Dauer des Effektes in Minuten an.

c) Δ HF (S/min): Änderung der Herzfrequenz in Schlägen pro Minute.

d) Δ dp/dt (mm Hg/s): Änderung der Druckanstiegsgeschwindigkeit in mm Hg pro Sekunde.

**Patentansprüche für die Vertragsstaaten: BE CH DE FR GB IT LI NL SE**

1. Substituiertes 3-Aminosydnonimin der Formel I

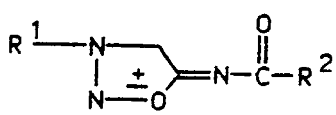

(I)

in optisch aktiver Form und seine pharmakologisch annehmbaren Säureadditionssalze, worin

$R^1$ den Rest

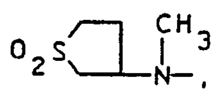

$R^2$ den Rest 2-Bornyloxy bedeuten.

2. Verfahren zur Herstellung der Verbindung des Anspruchs 1, dadurch gekennzeichnet, daß das Sydnonimin der Formel III

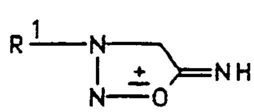

(III)

oder ein Salz davon, worin $R^1$ die in Anspruch 1 definierte Bedeutung besitzt, mit einem Acylierungsmittel, das den Acylrest $R^2$—CO-einführt, umgesetzt wird, wobei $R^2$ die im Anspruch 1 angegebene Bedeutung besitzt und die erhaltene Verbindung gegebenenfalls in an sich bekannter Weise in die optisch aktive Form und/oder in ein Säureadditionssalze überführt wird.

3. Verfahren nach Anspruch 2, dadurch gekennzeichnet, daß als Acylierungsmittel eine Verbindung der Formel IV

$$R^2—CO—X \qquad (IV)$$

worin $R^2$ die in Anspruch 1 angegebene Bedeutung besitzt und X=Halogen, —OH, —O-Alkyl, —O—CO—$R^2$, —O—CO—O-Alkyl, —O-Aryl, —O-Nitroaryl, —O-Dinitroaryl, —$OCH_2CN$ oder den über ein N-Atom

gebundenen Rest eines Azols oder Benzazols mit mindestens 2 N-Atomen im quasi-aromatischen Fünfring bedeutet, eingesetzt wird.

4. Verfahren nach Anspruch 2 und/oder 3, dadurch gekennzeichnet, daß das Acylierungsmittel in optisch aktiver Form eingesetzt wird.

5. 3-Amino-sydnonimin der Formel I des Anspruchs 1 oder ein pharmakologisch annehmbares Säureadditionssalz davon als pharmakologischer Wirkstoff zur Anwendung bei der Bekämpfung und Vorbeugung cardiovaskulärer Erkrankungen.

6. Pharmazeutisches Präparat, dadurch gekennzeichnet, daß es die Verbindung des Anspruchs 1 oder ein pharmakologisch annehmbares Säureadditionssalz davon als Wirkstoff zusammen mit pharmazeutisch annehmbaren Träger- und Zusatzstoffen und gegebenenfalls noch eine oder mehrere andere pharmakologische Wirkstoffe enthält.

**Patentansprüche für den Vertragsstaat: AT**

1. Verfahren zur Herstellung des substituierten 3-Aminosydnonimins der Formel I

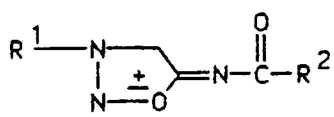

(I)

in optisch aktiver Form und seiner pharmakologisch annehmbaren Säureadditionssalze, worin

$R^1$ den Rest

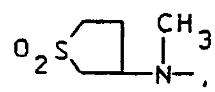

$R^2$ den Rest 2-Bornyloxy bedeuten, dadurch gekennzeichnet, daß das Sydnonimin der Formel III

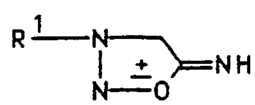

(III)

oder ein Salz davon, mit einem Acylierungsmittel, das den Acylrest $R^2$—CO-einführt, umgesetzt wird und die erhaltene Verbindung gegebenenfalls in an sich bekannter Weise in die optisch aktive Form und/oder in ein Säureadditionssalze überführt wird.

2. Verfahren nach Anspruch 1, dadurch gekennzeichnet, daß als Acylierungsmittel eine Verbindung der Formel IV

$$R^2—CO—X \qquad (IV)$$

worin X=Halogen, —OH, —O-Alkyl, —O—CO—$R^2$, —O—CO—O-Alkyl, —O-Aryl, —O-Nitroaryl, —O-Dinitroaryl, —$OCH_2CN$ oder den über ein N-Atom gebundenen Rest eines Azols oder Benzazols mit mindestens 2 N-Atomen im quasi-aromatischen Fünfring bedeutet, eingesetzt wird.

3. Verfahren nach Anspruch 2 und/oder 3, dadurch gekennzeichnet, daß das Acylierungsmittel in optisch aktiver Form eingesetzt wird.

4. Verfahren zur Herstellung pharmazeutischer Präparate zur Bekämpfung und Vorbeugung cardiovaskulärer Erkrankungen, dadurch gekennzeichnet, daß die nach einem oder mehreren der Ansprüche 1 bis 3 hergestellte Verbindung oder ein Pharmakologisch annehmbares Säureadditionssalz davon als Wirkstoff zusammen mit pharmazeutisch annehmbaren Träger- und Zusatzstoffen und gegebenenfalls noch einem oder mehrerer anderen pharmakologischen Wirkstoffen in an sich bekannter Weise in ein pharmazeutisches Präparat überführt wird.

**Revendications pour les Etats contractants: BE CH DE FR GB IT LI NL SE**

1. Amino-3 sydnonimine substituée répondant à la Formule I:

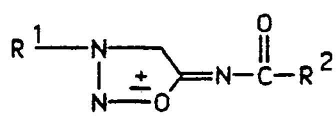

(I)

dans laquelle $R_1$ représente le radical:

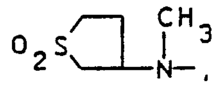 et

$R^2$ représente le radical bornyl-2 oxy, sous une forme optiquement active, et sels pharmacologiquement acceptables que ce composé forme par addition avec des acides.

2. Procédé pour préparer le composé de la revendication 1, procédé caractérisé en ce qu'on fait réagir la sydnonimine répondant à la Formule III:

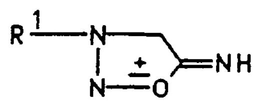

(III)

dans laquelle $R^1$ a la signification donnée à la revendication 1, ou un sel de celle-ci, avec un agent d'acylation introduisant le radical acyle $R^2$—CO— dans lequel $R^2$ a la signification qui lui a été donnée dans la revendication 1, et on transforme éventuellement le composé obtenu, de manière connue, en la forme optiquement active et/ou en un sel d'addition d'acide.

3. Procédé selon la revendication 2 caractérisé en ce qu'on utilise, comme agent d'acylation, un composé répondant à la Formule IV:

$$R^2\text{—CO—X} \qquad \text{(IV)}$$

dans laquelle $R^2$ a la signification qui lui a été donnée dans la revendication 1 et X représente un halogène, —OH, —O-alkyl, —O—CO—$R^2$, —O—CO—O-alkyl, —O-aryl, —O-nitro-aryl, —O-dinitro-aryl, —$OCH_2CN$ ou le radical, lié par un atome d'azote, d'un azole ou d'un benzazole à au moins 2 atomes d'azote dans un cycle pentagonal quasi-aromatique.

4. Procédé selon l'une des revendications 2 et 3, caractérisé en ce que l'agent d'acylation est mis en jeu sous une forme optiquement active.

5. Amino-3 sydnonimine de Formule I selon la revendication 1 ou sel d'addition d'acide, pharmacologiquement acceptable, d'un tel composé, en tant que substance active pharmacologique destinée à être utilisée pour le traitement curatif ou préventif de maladies cardio-vasculaires.

6. Composition pharmaceutique caractérisée en ce qu'elle contient, comme substance active, le composé selon la revendication 1, ou un sel d'addition d'acide, pharmacologiquement acceptable, d'un tel composé, associé à des excipients et additifs acceptables du point de vue pharmaceutique, et éventuellement une ou plusieurs autres substances douées d'une activité pharmacologique.

**Revendications pour l'Etat contractant: AT**

1. Procédé pour préparer l'amino-3 sydnonimine substituée repondant à la Formule I:

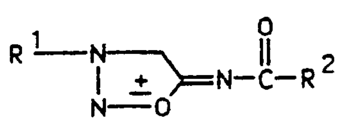

(I)

dans laquelle $R_1$ représente le radical:

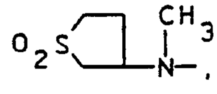 et

$R^2$ représente le radical bornyl-2 oxy, sous une forme optiquement active, et des sels pharmacologiquement acceptables que ce composé forme par addition avec des acides, procédé caractérisé en ce qu'on fait réagir a sydnonimine répondant à la Formule III:

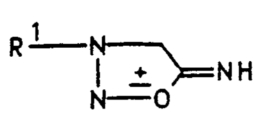

(III)

ou un sel d'addition de celle-ci, avec un agent d'acylation introduisant le radical acyle $R^2$—CO—, et on transforme éventuellement le composé obtenu, de manière connue, en une forme optiquement active et/

ou en un sel d'addition d'acide.

2. Procédé selon la revendication 1 caractérisé en ce qu'on utilise, comme agent d'acylation, un composé répondant à la Formule IV:

$$R^2—CO—X \quad (IV)$$

dans laquelle X représente un halogène, —OH, —O-alkyl, —O—CO—$R^2$, —O—CO—O-alkyl, —O-aryl, —O-nitro-aryl, —O-dinitro-aryl, —$OCH_2CN$ ou le radical, lié par un atome d'azote, d'un azole ou d'un benzazole à au moins 2 atomes d'azote dans un cycle pentagonal quasi-aromatique.

3. Procédé selon l'une des revendications 1 et 2, caractérisé en ce que l'agent d'acylation est mis en jeu sous une forme optiquement active.

4. Procédé pour préparer des compositions pharmaceutiques destinées à combattre et à prévenir des maladies cardio-vasculaires, procédé caractérisé en ce qu'on met sous la forme d'une composition pharmaceutique, de manière connue, le composé préparé selon l'une quelconque des revendications 1 à 3, ou un sel d'addition, pharmacologiquement acceptable, d'un tel composé, en tant que substance active, avec des excipients et additifs acceptables du point de vue pharmaceutique et, éventuellement, avec une ou plusieurs autres substances douées d'une activité pharmacologique.

**Claims for the Contracting States: BE CH DE FR GB IT LI NL SE**

1. Substituted 3-aminosydnonimine of the general formula I

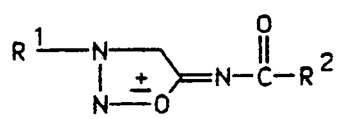

(I)

in optically active form, and its pharmacologically acceptable acid addition salts, wherein $R^1$ denotes the radical

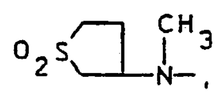

and $R^2$ denotes the radical 2-bornyloxy.

2. Process for the preparation of the compound of Claim 1, characterized in that the sydnonimine of the formula III

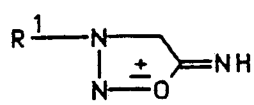

(III)

or a salt thereof, wherein $R^1$ has the meaning defined in Claim 1, is reacted with an acylating agent which introduces the acyl radical $R^2$—CO—, wherein $R^2$ has the meaning given in Claim 1, and, if appropriate, the resulting compound is converted into the optically active form and/or into an acid addition salt in a manner which is known per se.

3. Process according to Claim 2, characterized in that a compound of the formula IV

$$R^2—CO—X \quad (IV)$$

wherein $R^2$ has the meanings given in Claim 1 and X denotes halogen, —OH, —O-alkyl, —O—CO—$R^2$—, —O—CO—O-alkyl, O-aryl, O-nitroaryl, —O-dinitroaryl, —$OCH_2CN$ or the radical of an azole or benzazole which has at least 2 N atoms in the quasi-aromatic five-membered ring and is bonded via an N atom, is employed as the acylating agent.

4. A process according to claim 2 and/or 3, characterized in that the acylating agent is employed in optically active form.

5. 3-Amino-sydnonimine of the formula I of claim 1 or a pharmacologically acceptable acid addition salt thereof as pharmacological active compound for use in combating and preventing cardiovascular diseases.

6. Pharmaceutical product, characterized in that it contains the compound of Claim 1 or a pharmacologically acceptable acid addition salt thereof as the active compound, together with

pharmaceutically acceptable excipients and additives and, if appropriate, also one or more other pharmacological active compounds.

**Claims for the Contracting State: AT**

1. Process for preparing the substituted 3-aminosydnonimine of the general formula I

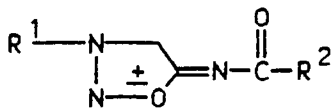

(I)

in optically active form, and its pharmacologically acceptable acid addition salts, wherein $R^1$ denotes the radical

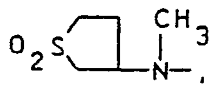

and $R^2$ denotes the radical 2-bornyloxy, characterized in that the sydnonimine of the formula III

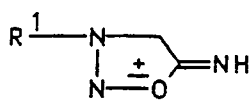

(III)

or a salt thereof, is reacted with an acylating agent which introduces the acyl radical $R^2$—CO— and, if appropriate, the resulting compound is converted into the optically active form and/or into an acid addition salt in a manner which is known per se.

2. Process according to Claim 1, characterized in that a compound of the formula IV

$$R^2\text{—CO—}X \quad (IV)$$

wherein X denotes halogen, —OH, —O-alkyl, —O—CO—$R^2$—, —O—CO—O-alkyl, O-aryl, O-nitroaryl, —O-dinitroaryl, —$OCH_2CN$ or the radical of an azole or benzazole which has at least 2 N atoms in the quasi-aromatic five-membered ring and is bonded via an N atom, is employed as the acylating agent.

3. Process according to Claim 1 and/or 2, characterized in that the acylating agent is employed in optically active form.

4. Process for preparing pharmaceutical products for use in combating and preventing cardiovascular diseases, characterized in that the compound prepared according to one or several of claims 1 to 3 or a pharmacologically acceptable acid addition salt thereof is converted, as active compound, in a manner known per se, together with pharmaceutically acceptable excipients and additives and, if appropriate, also one or more other pharmacological active compounds, into a pharmaceutical preparation.